# FASCICULE DE BREVET EUROPEEN

(11) **EP 1 307 242 B1**
(45) Date de publication et mention de la délivrance du brevet: **10.01.2007**
(21) Numéro de dépôt: 01963040.9
(22) Date de dépôt: 31.07.2001
(51) Int. Cl.: A61L 9/01, A61L 9/04, A61L 9/12

(54) **METHODE DE TRAITEMENT BACTERICIDE, FONGICIDE, VIRUCIDE ET INSECTICIDE DE L'AIR AMBIANT**
BAKTERIZIDE, FUNGIZIDE, VIRUZIDE UND INSEKTIZIDE BEHANDLUNGSMETHODE FÜR RAUMLUFT
METHOD FOR BACTERICIDAL, FUNGICIDAL, VIRUCIDAL AND INSECTICIDAL TREATMENT OF AMBIENT AIR

(30) Priorité: 31.07.2000 FR 0010064
(43) Date de publication de la demande: 07.05.2003
(73) Titulaire: Hightech Bio Activities - HBA, 38350 La Mure (FR)
(72) Inventeur: BELBACHIR, Hakima, F-38350 La Mure (FR); ANGELIDIS, Jean, F-38330 Saint Ismier (FR)
(74) Mandataire: Dorland, Anne-Marie
(86) Numéro de dépôt international: PCT/FR2001/002506
(87) Numéro de publication internationale: WO 2002/009777

(56) Documents cités:
- WO-A-97/35625
- FR-A- 2 603 806
- GB-A- 2 251 792
- GB-A- 2 259 858
- US-A- 3 547 576
- US-A- 5 246 919
- DATABASE WPI Section Ch, Week 199249 Derwent Publications Ltd., London, GB; Class D22, AN 1992-406001 XP002165110 & SU 1 702 111 A (PERM POLY), 30 décembre 1991 (1991-12-30) cité dans la demande

## Description

### Domaine technique de l'invention

L'invention est du domaine du traitement de l'air, et elle a pour objet une méthode pour appliquer un traitement bactéricide, fongicide, virucide et insecticide de l'air ambiant, ainsi qu'un dispositif pour la mise en oeuvre de cette méthode et les applications qui en sont faites.

### Etat de la technique

On connaît diverses méthodes de traitement physico-chimique de l'air ambiant. D'une manière générale, l'air est entraîné à travers un contenant perméable à l'intérieur duquel est placé au moins un agent traitant. Par exemple, le traitement de l'air étant un traitement mécanique visant à en retirer les poussières en suspension, l'agent traitant est un filtre constitué d'une feuille micro perforée, en matière cellulaire ou synthétique, ou encore d'une couche de matière alvéolaire. Par exemple encore, le traitement de l'air étant un traitement chimique, l'agent traitant est constitué d'un agent actif, tel qu'à base de charbon, pour modifier la composition chimique de l'air et/ou en absorber des gaz toxiques. Par exemple enfin, le traitement de l'air étant un traitement physico- chimique, l'agent traitant est une source de rayonnement, d'ultraviolets notamment. On comprendra que l'air peut être entraîné à travers plusieurs agents traitants successifs de nature respective, les contenants étant par exemple juxtaposés à l'intérieur d'un réceptacle commun comportant une admission d'air à traiter et une évacuation d'air traité.

Le document SU-A-1 702 111, décrit un dispositif de filtrage et de traitement de l'air vicié utilisant des cristaux de NaCl et KCl. L'air est ensuite refiltré avant d'être stérilisé par rayonnement au moyen d'une lampe.

Le problème à résoudre dans les dispositifs connus, est le risque de contamination du contenant suite à l'effet de filtrage mécanique.

### Objets de l'invention

Un objet de la présente invention est une méthode de traitement de l'air ambiant assurant la décontamination du contenant.

Un autre objet de l'invention est de proposer des moyens de mise en oeuvre de la méthode de traitement de l'air ambiant de l'invention dans le cadre d'applications domestiques.

La méthode selon la présente invention consiste à utiliser un agent traitant ayant des effets bactéricides, fongicides, virucides, et insecticides, en complément d'une filtration mécanique, et à répartir l'agent traitant uniformément à l'intérieur du contenant, de manière à empêcher le développement de micro-organismes sur le contenant lui même.

L'invention fournit une méthode de traitement de l'air ambiant, consistant à entraîner ou à faire circuler l'air à travers un contenant perméable à l'intérieur duquel est placé un agent traitant comprenant des cristaux d'au moins un sel minéral, caractérisée en ce que ledit agent traitant est utilisé en tant que bactéricide, fongicide, virucide et insecticide en complément d'une filtration mécanique de l'air et qu'il contient en outre des éléments d'origine végétale parcellisés choisis parmi le clou de girofle et le thym mélangés audit sel minéral, ledit agent traitant étant réparti uniformément à l'intérieur du contenant de manière à empêcher le développement de micro-organismes sur le contenant lui même.

Selon une disposition de l'invention, le sel minéral est du chlorure de sodium.

L'invention concerne aussi un dispositif de traitement de l'air ambiant, comportant deux feuilles micro-perforées de filtration mécanique de l'air, une couche de l'agent traitant bactéricide, fongicide, virucide et insecticide de l'invention, laquelle couche est répartie uniformément entre lesdites feuilles pour empêcher le développement de micro-organismes sur les parois. Grâce à ces dispositions, l'air ambiant, non seulement est traité mécaniquement pour en retirer les particules en suspension et biologiquement pour son assainissement, mais aussi est utilisé pour véhiculer des substances bénéfiques pour la santé des individus.

On comprendra que le contenant de l'agent traitant de l'invention est le cas échéant juxtaposable, dans le sens d'entraînement, ou de circulation, de l'air, avec au moins un autre contenant qui contient un agent traitant d'une autre nature, mécanique et/ou physico-chimique notamment, pour former un dispositif global de traitement de l'air ambiant.

Le mode de circulation de l'air ambiant à travers l'agent traitant de l'invention, ainsi que l'agencement du contenant, indiffèrent quant à la portée de l'invention et sont attachés à l'application spécifique qui en est faite.

Ainsi et selon diverses variantes du mode de circulation de l'air à travers l'agent traitant de l'invention, celui-ci est entraîné de manière artificielle ou de manière naturelle.

Selon une première variante, la circulation d'air est provoquée artificiellement par ventilation. Cette variante s'inscrit notamment dans le cadre d'un premier groupe d'applications de l'invention à des dispositifs d'échange calorique, tels que de chauffage ou frigorifiques, des climatiseurs, ou des appareils de recyclage de l'air.

Selon une deuxième variante, la circulation de l'air est provoquée naturellement, par évaporation de gaz ou par variation de pression par exemple. Cette variante s'inscrit notamment dans le cadre d'un deuxième groupe d'applications de l'invention à l'épuration d'air en provenance d'un milieu en fermentation, et plus particulièrement aux conteneurs de déchets organiques.

Ainsi encore et selon diverses variantes du contenant, notamment en correspondance avec les différents modes susvisés e circulation de l'air à travers l'agent traitant de l'invention, celui-ci peut être contenu à l'intérieur d'un boîtier perméable maintenu sur un support, ou encore être contenus dans un filet souple refermé sur lui-même pour former un sac, ou encore être contenus entre les parois d'un contenant souple à double paroi.

Selon une première variante du contenant, qui correspond notamment au premier groupe d'applications susvisées, le boîtier, ou alternativement le filet, est soit interchangeable, soit organisé de manière à permettre le remplacement de l'agent traitant. Par ailleurs, le boîtier, ou alternativement le filet, est le cas échéant disposé à l'intérieur d'un réceptacle comportant une admission d'air à traiter et une évacuation d'air traité. On comprendra que le contenant, boîtier ou alternativement filet, est accessoirement juxtaposé à au moins un contenant similaire d'un agent traitant mécanique, chimique ou physico-chimique des différents genres susvisés.

Selon une deuxième variante du contenant, correspondante au deuxième groupe d'applications susvisées, le contenant à double paroi est par exemple refermé sur lui-même pour former en outre un sac destiné à contenir des déchets, ménagers notamment. Par exemple encore, le contenant à double paroi est agencé en bâche de recouvrement d'un matériau fermentescible, pour fosse d'enfouissement ou conteneur de transformation pour déchets organiques notamment.

### Description sommaire des dessins

La présente invention sera mieux comprise et des détails en relevant apparaîtront, à la description qui va en être faite de formes préférées de réalisation et d'application, en relation avec les figures des planches annexées, dans lesquelles :
les fig.1 et fig.3 sont des représentations schématiques de différentes formes respectives de réalisation d'un dispositif de traitement de l'air ambiant, qui met en oeuvre une méthode de l'invention,
la fig.4 est une représentation schématique d'un dispositif de traitement de l'air ambiant selon une forme particulière d'application d'une méthode de l'invention, qui met en oeuvre divers agents traitant de nature respective,
les fig.5 à fig.9 sont des schémas illustrant divers exemples d'applications de la méthode de traitement de l'air ambiant proposée par l'invention.

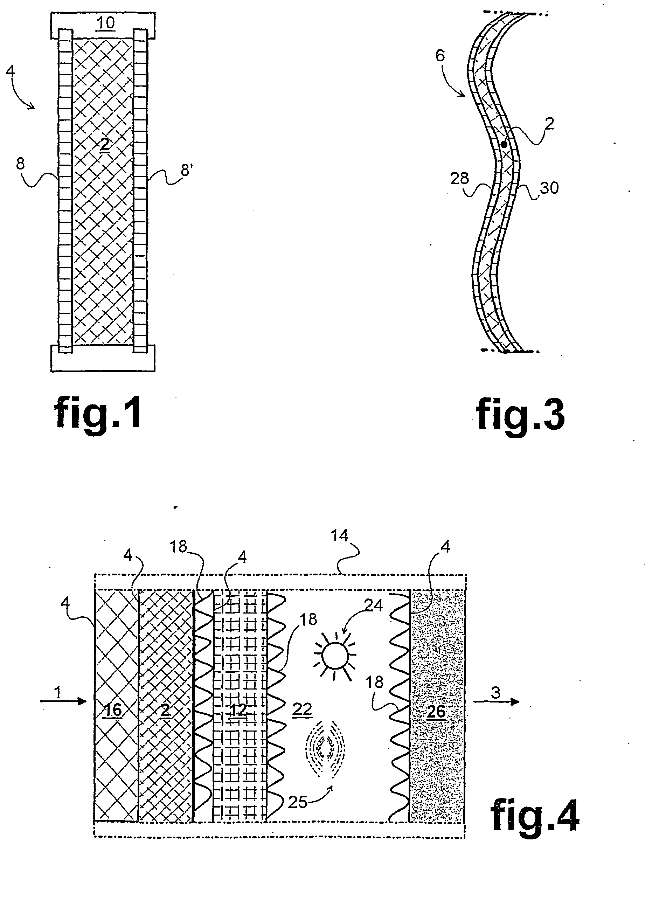

Sur les fig.1 et fig.3, un dispositif de traitement bactéricide, fongicide et/ou insecticide de l'air ambiant met en oeuvre l'agent traitant de l'invention 2. Sur les divers exemples de réalisation illustrés, l'agent traitant de l'invention 2 est réparti uniformément à l'intérieur d'un contenant perméable, tel que 4 ou 6, de manière à empêcher le développement de micro-organismes sur le contenant lui-même.

### Description de modes de réalisations préférentiels

Sur la fig.1 le contenant 4 est constitué par deux feuilles micro perforées 8,8' d'origine synthétique ou naturelle, et supportées par un cadre 10. On notera que le cadre 10 comporte de préférence une ouverture, non représentée sur les figures, permettant d'introduire les cristaux de sel entre les deux feuilles 8,8', voire de les remplacer après un usage prolongé. Ces dispositions sont telles que le contenant 4 est agencé en cassette de produit traitant 2, susceptible d'être supportée de manière amovible par un réceptacle, en vue de son interchangeabilité.

En se reportant plus particulièrement sur la fig.4, un dispositif de traitement global de l'air comporte un réceptacle 14 à plusieurs compartiments, qui logent chacun de manière amovible un contenant 4 d'un agent traitant de nature et/ou d'origine respectives.

Un tel dispositif comprend de préférence l'un quelconque au moins des agents traitant successifs suivants:
- une strate de mousse 16, telle que ouate naturelle ou synthétique, pour une première filtration mécanique, grossière, de l'air pollué 1,
- une strate d'agent traitant de l'invention 2 pour un traitement bactéricide, virucide et/ou fongicide de l'air,
- un filtre mécanique fin 18, tel que filtre de papier ou de tissu,
- une strate de végétaux spécifiques parcellisés 12, tel que thym et/ou clous de girofle, pour compléter l'action bactéricide et/ou fongicide et/ou insecticide des cristaux de sel minéraux;
- un filtre mécanique fin 18 du type susvisé,
- un compartiment 22 de traitement de l'air par rayonnement, tel que par rayons ultraviolets 24 et/ou par ondes magnétiques 25 et/ou par ondes sonores, pour un traitement germicide de l'air,
- un filtre mécanique fin 18 du type susvisé,
- une strate de charbon actif 26 et/ou de laine naturelle, et:ou de terres rares pour éventuellement absorber les gaz toxiques.

On comprendra que les divers agents traitants 16,2,12,26 sont logés dans des contenants semblables respectifs, en vue de leur interchangeabilité ou de leur retrait selon le, ou les, traitements de l'air souhaités par l'utilisateur.

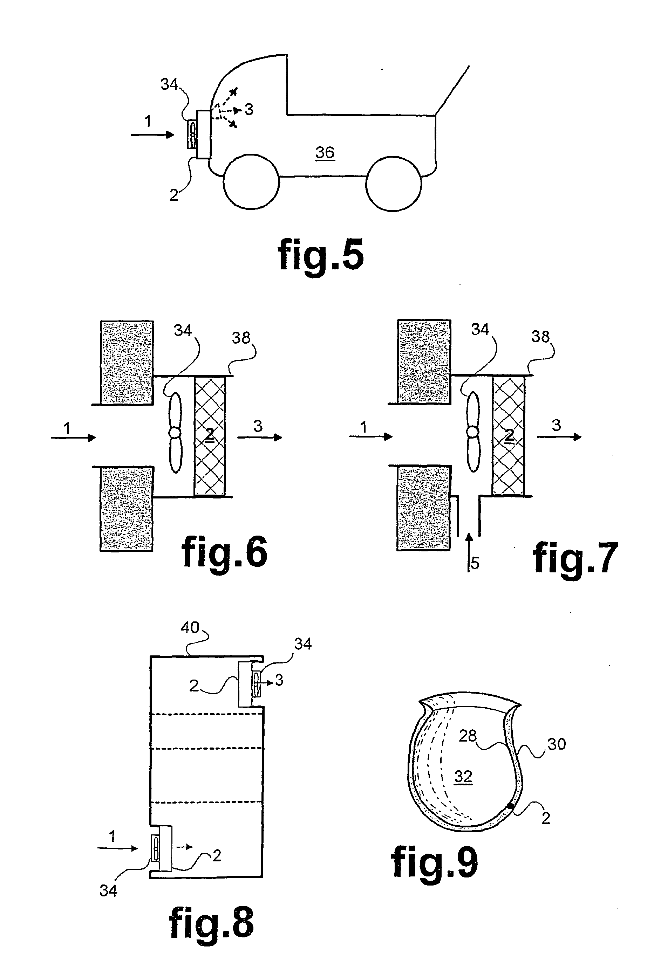

Sur la fig.3, le contenant 6 est constitué par une feuille souple micro-perforée à double paroi 28 et 30, en vue par exemple de la confection d'un sac à déchets ménagers 32, tel que celui illustré sur la fig.9.

Sur les fig.5 à fig.8, un dispositif mettant en oeuvre la méthode de l'invention comprend en outre des moyens 34 de circulation par ventilation de l'air à traiter à travers au moins un agent traitant, et plus particulièrement à travers au moins un agent traitant de l'invention 2.

Sur la fig.5, la méthode de l'invention est appliquée à un chariot 36 pour enfant. On notera des applications analogues, telles qu'à l'habitacle d'un véhicule ou à un casque de sécurité.

Sur les fig.6 et 7, la méthode de l'invention est appliquée aux installations ou aux appareils échangeurs de calories qui comportent un circuit 38 de ventilation d'air, tels que climatiseurs, radiateurs ou réfrigérateurs (fig.6) ou encore aux appareils ou aux installations de recyclage d'air (fig.7). On notera que selon cette dernière application, le dispositif peut comporter non seulement une admission d'air pollué 1, mais aussi une admission annexe d'air extérieur 5.

Sur la fig.8, la méthode de l'invention est appliquée à l'assainissement des milieux clos, notamment des placards à chaussures 40, chambres froides, appareils frigorifiques, ustensiles pour les bébés, etc....

On relèvera aussi dans le domaine du bâtiment une application de l'invention, non représentée sur les figures, aux éléments de revêtement de surface, telle que sol, mur ou plafond, pour limiter le développement de bactéries ou de champignons. Selon cette application, le contenant de l'agent traitant le l'invention est par exemple agencé en panneau ou en film à double paroi.

## Revendications

1. Méthode de traitement de l'air ambiant, consistant à entraîner ou à faire circuler l'air (1) à travers un contenant (4, 6) perméable à l'intérieur duquel est placé un agent traitant (2) comprenant des cristaux d'au moins un sel minéral, **caractérisée en ce que** ledit agent traitant est utilisé en tant que bactéricide, fongicide, virucide et insecticide en complément d'une filtration mécanique de l'air et qu'il contient en outre des éléments d'origine végétale parcellisés choisis parmi le clou de girofle et le thym mélangés audit sel minéral, ledit agent traitant étant réparti uniformément à l'intérieur du contenant (4, 6) de manière à empêcher le développement de micro-organismes sur le contenant lui même.

2. Méthode selon la revendication 1, **caractérisée en ce que** le sel minéral est du chlorure de sodium.

3. Dispositif pour la mise en oeuvre d'une méthode selon la revendication 1, **caractérisé en ce qu'**il comprend, entre deux feuilles micro-perforées (8, 8') de filtration mécanique de l'air, une couche d'agent traitant (2) bactéricide, fongicide, virucide et insecticide comprenant des cristaux de sel minéral mélangés à des agents traitants d'origine végétale parcellisés choisis parmi le clou de girofle et le thym, ladite couche étant répartie uniformément entre lesdites feuilles (8, 8') pour empêcher le développement de micro-organismes sur les parois.

4. Dispositif selon la revendication 3, **caractérisé en ce que** le contenant est l'un quelconque des contenants du groupe de contenants comprenant un boîtier (4), un filet et un contenant souple (6) à double paroi.

5. Dispositif selon la revendication 3, **caractérisé en ce qu'**il comprend des moyens de circulation d'air par ventilation (34) à travers le contenant.

6. Dispositif selon la revendication 3, **caractérisé en ce qu'**il comprend l'un quelconque au moins des agents traitants successifs suivants :
- une strate de mousse (16) pour une première filtration mécanique,
- une strate (2) de cristaux de sels minéraux mélangés à agents traitants d'origine végétale parcellisés choisis parmi le clou de girofle et le thym,
- un filtre mécanique fin (18)
- un compartiment (22) de traitement de l'air par rayonnement (24, 25)
- un filtre mécanique fin (18)
- une strate de charbon actif (26).

7. Application d'une méthode selon la revendication 1 aux dispositifs d'échange calorique du groupe des dispositifs (38) comprenant les climatiseurs, les appareils de chauffage, les appareils frigorifiques et les appareils de ventilation et de recyclage de l'air.

8. Application d'une méthode selon la revendication 1 aux conteneurs de déchets organiques du groupe de conteneurs comprenant les sacs de déchets ménagers (32), les fosses d'enfouissement de matériau fermentescible et les conteneurs de transformation de déchets organiques.

9. Application d'une méthode selon la revendication 1 aux éléments de revêtement de surface d'un bâtiment.

## Claims

1. A method for treating ambient air, consisting in drawing or in circulating the air (1) through a permeable container (4, 6) in which is placed a treating agent (2) comprising crystals of at least a mineral salt, **characterized in that** said treating agent is used as a bactericide, fungicide, virucidal and insecticide, in addition to mechanical filtration of the air, and **in that** it further contains fragmented elements of plant origin of the group of clove and thyme mixed to said mineral salt, said treating agent being distributed uniformly inside the container (4, 6), so as to prevent the development of microorganisms on the container itself.

2. The method as claimed in claim 1, **characterized in that** the mineral salt is sodium chloride.

3. A device for implementing the method as claimed in claim 1, **characterized in that** it comprises, between two microperforated sheets (8, 8') for mechanical filtration of the air, a layer of bactericidal, fungicidal, virucidal, and insecticidal treating agent (2) comprising crystals of mineral salt mixed to fragmented elements of plant origin of the group of clove and thyme, said layer being distributed uniformly between said sheets (8, 8') so as to prevent the development of microorganisms on the walls.

4. The device as claimed in claim 3, **characterized in that** the container is anyone of the containers of the group of containers comprising a case (4), a net and a double-walled flexible container (6).

5. The device as claimed in claim 3, **characterized in that** it comprises means for circulating air through the container by ventilation (34).

6. The device as claimed in claim 3, **characterized in that** it comprises at least any one of the following successive treating agents:
- a layer of foam (16) for a first mechanical filtration,
- a layer (2) of mineral salt crystals mixed to fragmented elements of plant origin of the group of clove and thyme,
- a fine mechanical filter (18),
- a compartment (22) for treatment of air by radiation (24, 25),
- a fine mechanical filter (18),
- a layer of active charcoal (26).

7. The application of a method as claimed in claim 1, to calorie-exchanging devices of the group of devices (38) comprising air conditioners, heating appliances, refrigerating appliances, and ventilation and air-recycling appliances.

8. The application of a method as claimed in claim 1, to organic waste containers of the group of containers comprising household waste sacks (32), landfill pits for fermentable material and transformation containers for organic waste.

9. The application of a method as claimed in claim 1, to elements for coating a building surface.

## Patentansprüche

1. Verfahren zur Behandlung von Umgebungsluft, bei dem man die Luft (1) durch ein durchlässiges Behältnis (4, 6) treibt oder zirkuliert, in dem ein Behandlungsmittel (2) platziert ist, das Kristalle mindestens eines Mineralsalzes umfasst, **dadurch gekennzeichnet, dass** das Behandlungsmittel als Bakterizid, Fungizid, Viruzid und Insektizid zusätzlich zu einer mechanischen Filterung der Luft verwendet wird und dass es des Weiteren zerkleinerte Elemente pflanzlichen Ursprungs enthält, die unter einer Gewürznelke und Thymian, die mit dem Mineralsalz vermischt sind, ausgewählt sind, wobei das Behandlungsmittel im Inneren des Behältnisses (4, 6) gleichmäßig verteilt ist, um die Erzeugung von Mikroorganismen am Behältnis selbst zu verhindern.

2. Verfahren nach Anspruch 1, **dadurch gekennzeichnet, dass** das Mineralsalz Natriumchlorid ist.

3. Vorrichtung zur Durchführung eines Verfahrens nach Anspruch 1, **dadurch gekennzeichnet, dass** sie zwischen zwei mikroperforierten Flächengebilden (8, 8') zur mechanischen Filterung von Luft eine Lage Bakterizid-, Fungizid-, Viruzid- und Insektizid-Behandlungsmittel (2) mit Mineralsalzkristallen, vermischt mit zerkleinerten Behandlungsmitteln pflanzlichen Ursprungs, ausgewählt unter einer Gewürznelke und Thymian, umfasst, wobei die Lage zwischen den Flächengebilden (8, 8') gleichmäßig verteilt ist, um die Erzeugung von Mikroorganismen an den Wänden zu verhindern.

4. Vorrichtung nach Anspruch 3, **dadurch gekennzeichnet, dass** das Behältnis ein beliebiges unter Behältnissen einer ein Gehäuse (4), ein Netz und ein flexibles Behältnis (6) mit Doppelwand umfassenden Behältnisgruppe ist.

5. Vorrichtung nach Anspruch 3, **dadurch gekennzeichnet, dass** sie Mittel zur Zirkulation von Luft durch Ventilation (34) durch das Behältnis umfasst.

6. Vorrichtung nach Anspruch 3, **dadurch gekennzeichnet, dass** sie mindestens eines der folgenden aufeinander folgenden Behandlungsmittel umfasst:
- eine Schaumstoffschicht (16) für eine erste mechanische Filtration,
- eine Schicht (2) aus Mineralsalzkristallen, die mit zerkleinerten Behandlungsmitteln pflanzlichen Ursprungs, ausgewählt unter einer Gewürznelke und Thymian, vermischt sind,
- einen mechanischen Feinfilter (18),
- eine Kammer (22) zur Behandlung von Luft durch Strahlung (24, 25),
- einen mechanischen Feinfilter (18),
- eine Aktivkohleschicht (26).

7. Anwendung eines Verfahrens nach Anspruch 1 auf Wärmeaustauschvorrichtungen der Gruppe von Vorrichtungen (38), die Klimaanlagen, Heizvorrichtungen, Kühlvorrichtungen und Lüftungs-und Luftumwälzgeräte umfasst.

8. Anwendung eines Verfahrens nach Anspruch 1 auf Behältnisse für organische Abfälle der Gruppe von Behältnissen, die Haushaltsmüllsäcke (32), Deponiegruben für fermentierbares Material und Behältnisse zur Umwandlung von organischen Abfällen umfassen.

9. Anwendung eines Verfahrens nach Anspruch 1 auf Elemente zur Beschichtung einer Gebäudefläche.
